# EUROPEAN PATENT APPLICATION

(11) **EP 2 332 916 A2**
(43) Date of publication of application: **15.06.2011**
(21) Application number: 10156733.7
(22) Date of filing: 17.03.2010
(51) Int. Cl.: C07D 215/58

(54) **A process for a preparation of marbofloxacin and intermediate thereof**

(30) Priority: 19.11.2009 SI 200900366
(71) Applicant: Krka Tovarna Zdravil, D.D., Novo Mesto, 8501 Novo Mesto (SI)
(72) Inventor: Zupet, Rok, 1000, Ljubljana (SI); Pecavar, Anica, Novo mesto, 8000 (SI); Iskra, Jernej, 1000, Ljubljana (SI); Ruzic, Milos, 3000, Celje (SI); Kolenc, Ivanka, 8000, Novo mesto (SI)
(74) Representative: UEXKÜLL & STOLBERG

(57) **Abstract**

The present invention describes a novel process for the preparation of marbofloxacin and intermediate thereof comprising reaction of ammonium hydroxide of formula III, NR¹R²R³R⁴ wherein R¹, R², R³ and R⁴ are independently selected from the group of H, alkyl, alkylaryl, aryl and/or heteroaryl, with compound of formula II, 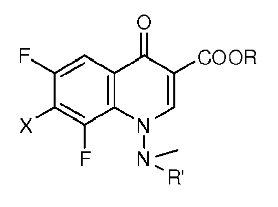 wherein R is selected from H, alkyl, arylalkyl, alkali metal cation, , NH₄ cation, NR¹R²R³R⁴cation; X is halogen, such as chloro, bromo, fluoro, piperazinyl, which may be substituted or unsubstituted, and R' is selected from H, formyl or COOAlkyl.

## Description

### Field of the invention

The present invention relates to a process for the preparation of intermediate of marbofloxacin and derivatives thereof, as well as to a process for the preparation of marbofloxacin and pharmaceutical formulations containing it.

### Background of the invention

Marbofloxacin is the common name for 9-fluoro-2,3-dihydro-3-methyl-10-(4-methyl-1-piperazinyl)-7-oxo-7H-pyridol(3,2,1-ij)(4,2,1)benzoxadiazin-6-carboxylic acid, of the formula :

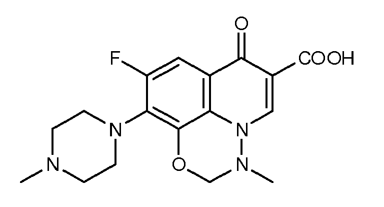

Marbofloxacin is a potent antibiotic of the fluoroquinolone group.

EP 259804 describes marbofloxacin as well as a synthesis for the preparation thereof by a multistep process which is unpractical for a large scale manufacture, since it requires high temperatures and reagents not suitable for large-scale production, resulting in low over-all yields. The process for the preparation is disclosed in the reaction scheme 1.

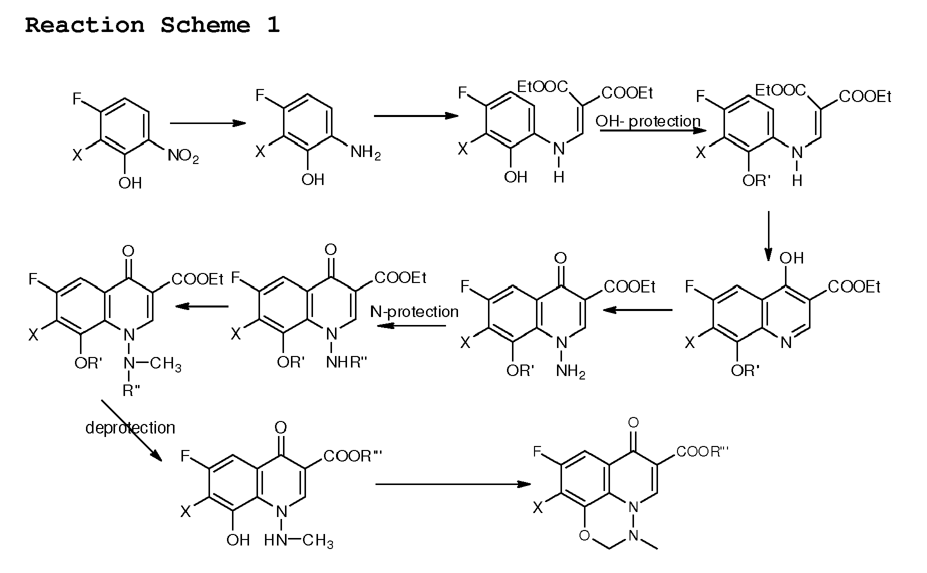

EP 680482 discloses an alternative approach for the preparation of marbofloxacin, wherein hydroxy group is introduced into molecule by means of reaction of intermediate with alkali metal hydroxide in aqueous media. The starting material used is 2,3,4,5-tetrafluorobenzoic acid. Disadvantages of this process are relatively high excess of alkali metal hydroxide and lengthy procedure. The process for the synthesis according to this patent is shown in the reaction scheme 2.

Research Disclosure No. 291, 1988, pages 548-551 discloses an alternative route of synthesis also starting from 2,3,4,5-tetrafluorobenzoic acid. Later steps of the process are shown in the reaction scheme 3.

IT 1313683 relates to a process for preparation of marbofloxacin by a process via benzyl ether. Ether was debenzylated in aqueous solution by hydrogenating over 5% Pd/BaSO₄ and the obtained product is cyclized using HCOOH/HCOH.

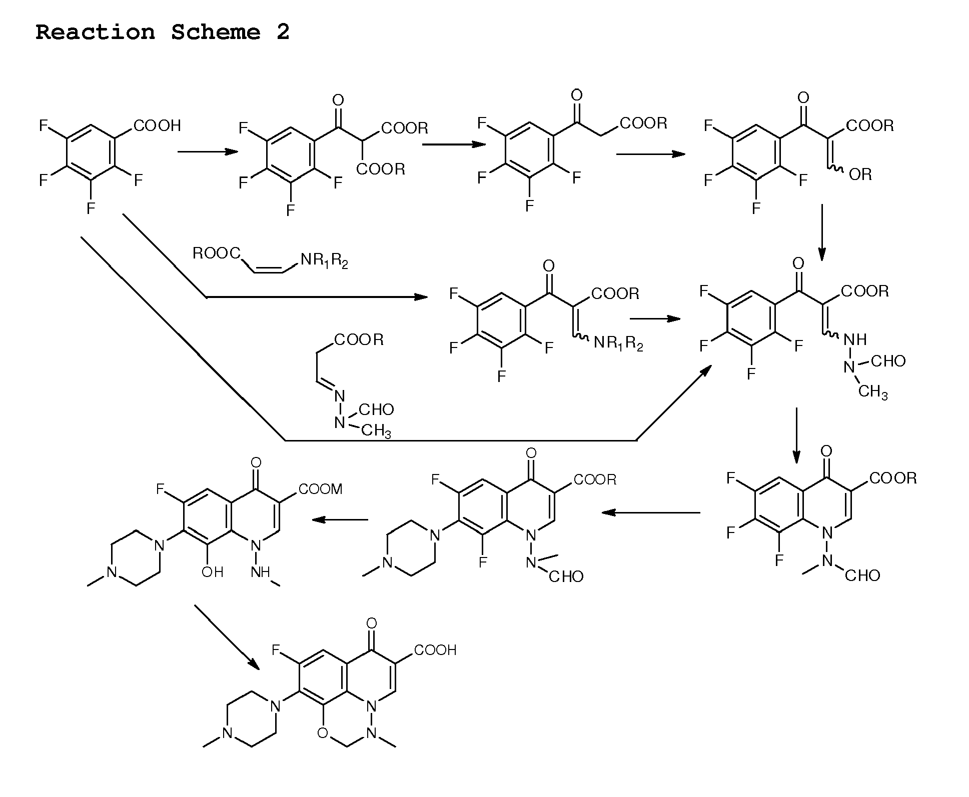

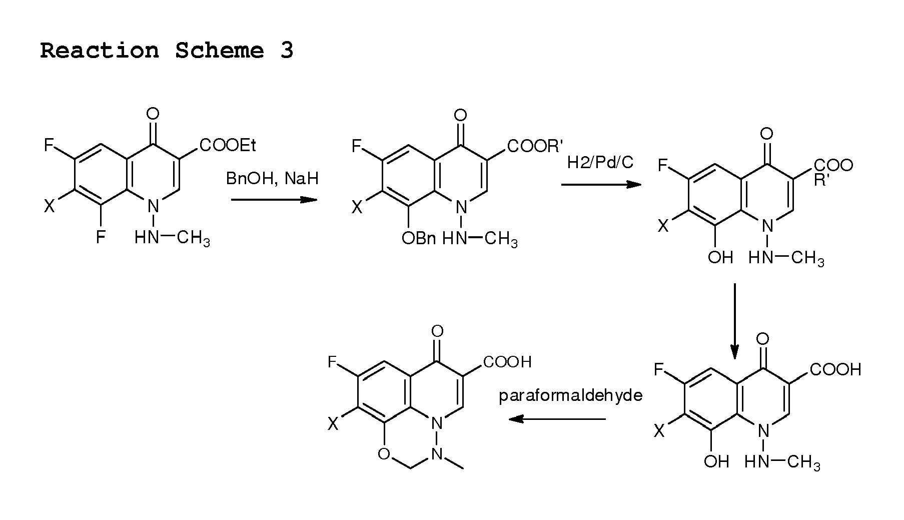

In view of the prior art there still exists a need for an improved method for preparation of marbofloxacin and intermediates thereof suitable for a large-scale production.

### Object of the invention

An object of the present invention resides in the provision of a new process for preparation of marbofloxacin intermediate and use thereof for the preparation of marbofloxacin and derivatives thereof.

According to a first embodiment of the present invention a process for the preparation of a compound with formula I is provided 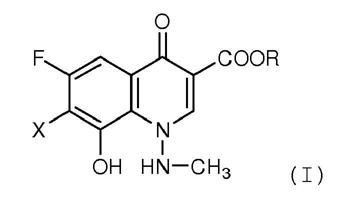 wherein
R is hydrogen, alkyl, arylalkyl, alkali metal cation, NH₄ cation, NR¹R²R³R⁴ cation,
R¹, R² R³ and R⁴ are independently selected from the group of H, alkyl, alkylaryl, aryl and/or heteroaryl, and
X is halogen, such as chloro, bromo, fluoro; piperazinyl, which is substituted or unsubstituted, such as 4-methyl piperazinyl,
which is characterized by reacting a compound of formula II, 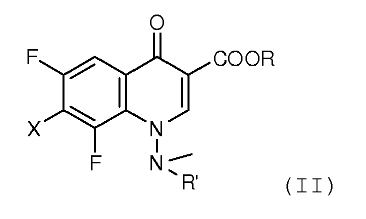
wherein R and X are as specified above, and
R' is selected from H, formyl, COOAlkyl,
with ammonium hydroxide of formula III,

R¹R²R³R⁴NOH (III)

wherein R¹, R², R³ and R⁴ are as specified above.

According to second embodiment of the invention a process for the one pot preparation of marbofloxacin or derivative thereof of formula IV is provided, 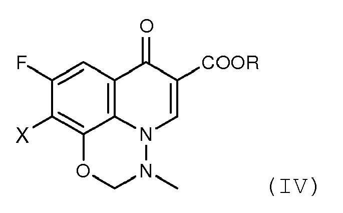 wherein R is H and X is piperazinyl, which may be substituted or unsubstituted, preferably 4-methyl piperazinyl, comprising the steps of
a) reacting compound of formula II, 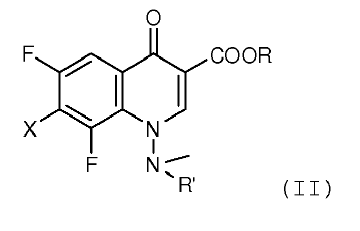
   wherein R is selected from H, alkyl, arylalkyl, alkali metal cation, NH₄ cation, NR¹R²R³R⁴ cation,
   X is selected from halogen, such as chloro, bromo or fluoro, piperazinyl, which may be substituted or unsubstituted, preferably 4-methyl piperazinyl,
   R' is selected from H, formyl, COOAlkyl,
   with ammonium hydroxide of formula III,

   R¹R²R³R⁴NOH (III)

   wherein R¹, R², R³ and R⁴ are as specified above, and
b) reacting the obtained intermediate in the same reactor with formic acid and formaldehyde, and optionally,
c) in case X is halogen its substitution by corresponding piperazine by conventional means to obtain after recovery compound of formula IV.

### Detailed description of the invention

According to a first embodiment of the present invention a process for the preparation of a compound with formula I is provided 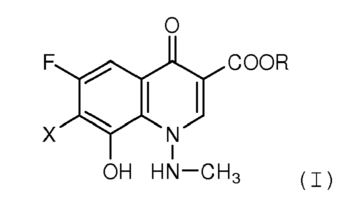 wherein
R is H, alkyl, arylalkyl, alkali metal cation, NH₄ cation, NR¹R²R³R⁴ cation,
R¹, R² R3 and R⁴ are independently selected from the group of H, alkyl, alkylaryl, aryl and/or heteroaryl, and
X is halogen, such as chloro, bromo, fluoro; piperazinyl, which may be substituted or unsubstituted, preferably 4-methyl piperazinyl,
which is characterized by reacting compound of formula II, 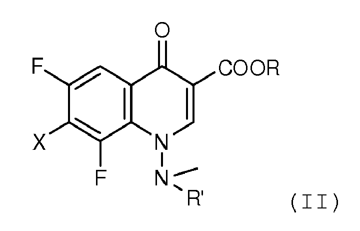
wherein R and X are as specified above and
R' is selected from H, formyl, COOAlkyl
with ammonium hydroxide of formula III,

R¹R²R³R⁴NOH (III)

wherein R¹, R², R³, R⁴ is as defined above.

Unless specifically limited otherwise, the term "alkyl", as well as derivative terms such as "arylalkyl", as used herein, include within their scope straight chain, branched chain and cyclic moieties. Unless specifically stated otherwise, each may be unsubstituted or substituted with one or more substituents selected from but not limited to halogen, hydroxy, alkoxy or alkylthio, provided that the substituents are sterically compatible and the rules of chemical bonding and strain energy are satisfied. The term "aryl" refers to a phenyl, indanyl or naphthyl group. The aryl group may be unsubstituted or substituted with one or more substituents selected from halogen, hydroxy, C₁-C₆ alkyl or C₁-C₆ alkoxy, provided that the substituents are sterically compatible and the rules of chemical bonding and strain energy are satisfied. The term "arylalkyl" refers to C₁-C₄ alkyl groups substituted with an aryl group. The heteroaryl group may be selected from a group consisting of pyridyl, thienyl, furyl, pyridazinyl, pyrimidinyl, pyrazinyl, thiazolyl and oxazolyl. The heteroaryl group may be unsubstituted or substituted with one or more substituents selected from halogen, hydroxy, C₁-C₆ alkyl or C₁-C₆ alkoxy, provided that the substituents are sterically compatible and the rules of chemical bonding and strain energy are satisfied.

Preferably the term alkyl refers to a straight and branched chain saturated aliphatic hydrocarbon radical having from 1 to 6 carbon atoms, such as methyl, ethyl, propyl, isopropyl, tert-butyl, or to a straight or branched chain arylakyl, such as benzyl, triphenylmethyl.

Preferably the compound of formula I and/or II according to present invention refers to a compound wherein R is selected from the group consisting of H, methyl, ethyl, alkali metal cation or NR¹R²R³R⁴; X is preferably selected from 4-methylpiperazine and R' is preferably selected from H.

The preferred ammonium hydroxide of formula III according to the first aspect of the invention refers to the compound wherein R¹, R², R³ and R⁴ are independently selected from the group H, methyl, ethyl, n- and i-propyl, n-butyl, benzyl, more preferred are those where R¹, R², R³ and R⁴ are the same or where R¹, R² and R³ are the same and R⁴ represents (C₂-C₁₆)alkyl, arylalkyl, most preferred are tetramethylammonium hydroxide and tetraethylammonium hydroxide.

According to the first embodiment of the invention, a process for the preparation of compound of formula I is preferred, wherein R and R' is H and X is 4-methylpiperazinyl, is provided wherein compound of formula II, wherein R is H or ethyl, R' is H and X is 4-piperazinyl, is reacted with tetramethyl ammonium hydroxide.

The reaction can be performed in neat conditions, in the absence of solvent, or in the presence of a solvent, or solvent mixtures.

In case the reaction is performed in the absence of a solvent ammonium hydroxide of formula III is preferably selected from hydrated form such as pentahydrate, 7.5 hydrate, decahydrate. It was found that the molten phase obtained by use of the hydrated form of compound III is easier to handle since it is less viscous . But the reaction can also be carried out with pure compound III without any solvent.

Alternatively, aqueous solution of ammonium hydroxide of formula III could be used. In this case the reaction mixture is heated to 100°C and water is left to evaporate until the oily molten phase is formed and then the reaction is further proceeded.

If an aqueous solution of compound III is used, it is preferred to remove excess water by evaporation, e.g. by distillation, preferably by vacuum (reduced pressure) distillation. If distillation is performed under normal pressure the reaction time required to complete the reaction was found to be above 20 hours, in case vacuum distillation is used, the reaction time can be reduced to 3 to 5 hours. Vacuum distillation is preferably performed at a temperature within the rage of 60 to 100°C, more preferably at about 80 to 100°C. The water is preferably removed to such an extend that the remaining quantity of water corresponds to 3 to 5 mole of water per mole of ammonium hydroxide of formula III. After the evaporation of excess water the reaction is preferably continued at elevated temperature, more preferably at a temperature of about 100°C.

In case the solvent is used it is selected from water or organic solvent such as polar or non polar organic solvent. The organic solvent can be selected from DMSO, THF, methyl tetrahydrofuran, ethylene glycol, polyethylene glycols, such as PEG300, PEG400, PEG500. Preferred solvents are DMSO, THF, methyl tetrahydrofuran, ethylene glycol, and polyethylene glycol.

In case organic solvent is used the presence of water is not desired, and compound III is preferably used in nonhydrated form. Furthermore, organic solvents and in particular hygroscopic solvents, such as DMF, DMSO, are preferably dried prior to use, e.g. by removing water dissolved in the solvent by distillation.

The reaction can be performed in the absence or in the presence of phase transfer catalyst, selected from crown ethers, or quaternary ammonium salts.

The phase transfer catalyst can be selected from the group of tetrabutylammonium chloride, tetrabutylammonium cyanide, tetrabutylammonium fluoride, tetrabutylammonium iodide, tetrabutylammonium hydroxide, tetrabutylphosphonium chloride, tricaprylylmethylammonium chloride, tetraethylammonium chloride, tetramethylammonium bromide, trioctylethylphosphonium bromide, trioctylmethylammonium chloride, trioctylpropylammonium chloride, tetrapropylammonium bromide, tetraphenylarsonium chloride, tetraphenylphosphonium bromide, tetraphenylphosphonium chloride, benzyltrimethylammonium hydroxide, 18-crown-6, dibenzo-18-crown-6, dicyclohexyl-18-crown-6 or mixtures thereof. Preferably, tetra-n-butylammonium bromide or tetra-n-butylammonium iodide is used.

Alternatively organic solvent can be selected from the group of ionic liquids.

Ionic liquids can be selected from the group 1-R-3-R1-imidazolium H(+) x A(-), wherein R and R1 are lower alkyl and A(-) are mono- or polyprotic anion of acid. The term lower alkyl is directed to C₁ to C₁₀ hydrocarbons, which may comprise linear and/or branched chains, saturated and/or unsaturated atoms, and heteroatoms.

Examples for suitable ionic liquids are 1,2,3-Trimethylimidazolium methylsulfate, 1-Butyl- 2,3-dimethylimidazolium chloride, 1-Butyl-3-methylimidazolium acetate, 1-butyl-3-methyl- imidazolium bromide, 1-Butyl-3-methylimidazolium hexafluorophosphate, 1-Butyl-3-methylimidazolium hydrogensulfate, 1-Butyl-3-methyl- imidazolium methansulfonate, 1-Butyl-3-methylimidazolium methylsulfate, 1-Butyl-3- methylimidazolium tetrachloroaluminate, 1-Butyl-3-methylimidazolium tetrafluoroborate, 1- Buty 1-3 -methylimidazolium thiocyanate, 1-Ethyl-2,3-dimethylimidazolium ethylsulfate, 1- Ethyl-3-methylimidazolium acetate, 1-Ethy 1-3 -methylimidazolium chloride, 1-Ethyl-3- methylimidazolium ethylsulfate, 1-Ethyl-3-methylimidazolium hydrogensulfate, 1-Ethyl-3-methylimidazolium methanesulfonate, 1-Ethyl-3-methylimidazolium methylsulfate, 1-Ethyl- 3-methylimidazolium tetrachloroaluminate, 1-Ethyl-3-methylimidazolium tetrafluoroborate, 1-Ethyl-3-methylimidazolium thiocyanate, 1-Hexyl-3 -methylimidazolium tetrafluoroborate, 1-Hexyl-3-methylimidazolium tetrafluoroborate, 1-Hexyl-3 -methylimidazolium trifluoro- methanesulfonate, 1-Methyl-3-octylimidazolium hexafluorophosphate, 1-Methyl-3-octyl-imidazolium trifluoromethanesulfonate, Methylimidazolium chloride, Methylimidazolium hydrogensulfate, Methyl-tri-n-butylammonium methylsulfate. Preferably 1-Ethyl-3-methyl- imidazolium ethylsulfate or 1-Ethyl-3-methylimidazolium methylsulfate are employed. Ionic liquids may be obtained for example by Merck, Germany. Also mixtures of one or more ionic liquids with water can be used.

The ratio of compound of formula II to ammonium hydroxide of formula III is from 1:1 to 1:10, preferably 1:3 to 1:7, most preferably 1:4 to 1:6.

The reaction may be carried out in the presence of a mineral base, more preferably in the presence of alkali metal carbonate or hydrogen carbonate, such as Na₂CO₃, Na-HCO₃, K₂CO₃, KHCO₃.

Reaction time and temperature of the reaction may be chosen according to common knowledge. Preferably the reaction is conduced for 1-20 hours at temperatures in the range of 60-120°C. More preferably, the reaction is conducted at a temperature from 80-110° C, for 4-8 hours.

After performing the reaction the mixture obtained is cooled, preferably to room temperature, and the excess hydroxide is neutralised by addition of acid. Examples of suitable acids that may be used include inorganic acids such as hydrogen chloride, phosphoric acid, and sulphuric acid; and organic acids such as formic acid, acetic acid, p-toluenesulphonic acid, methanesulphonic acid and trifluoroacetic acid.

The product is recovered as a free base or in the form of salt by addition of water or organic solvent or mixtures thereof. In a preferred embodiment organic solvents are selected from C₁ - C₅ alcohol, acetonitrile or tetrahydrofuran, methyl tetrahydrofuran, acetic acid, formic acid, acetone and/or ethers. More preferably alcohols are selected from methanol, ethanol and isopropanol and ethers are selected from tetrahydrofuran, methyl tetrahydrofuran, methyl t-butyl ether, diisopropyl ether.

Marbofloxacin or its derivative of formula IV 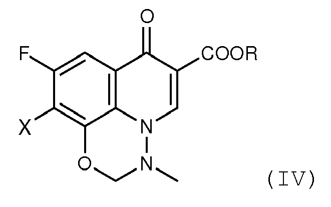 wherein R is H and X is piperaziniyl, which may be substituted or unsubstituted, preferably 4-methyl piperazinyl is prepared by cyclising compound of formula I, prepared in accordance with the first aspect of the present invention, with formic acid and formaldehyde followed by treatment with aqueous base, such as aqueous sodium hydroxide or aqueous ammonia. In case X is halogen it is substituted with suitable piperazine by means of methods known in the art. For the conversion of compound I into compound IV the same reaction conditions are used which are applied in the one pot process described in the following.

Second embodiment of the invention is a one pot process for the preparation of marbofloxacin or derivative thereof of formula IV 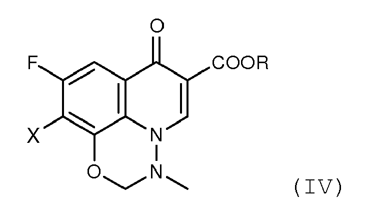 wherein R is H and X is piperazinyl, which may be substituted or unsubstituted, preferably 4-methyl piperazinyl,
comprising the steps of
a) reacting a compound of formula II 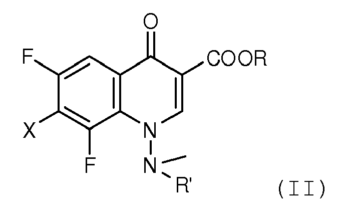
   wherein R is selected from H, alkyl, arylalkyl, alkali metal cation, NH₄ cation, NR¹R²R³R⁴ cation,
   R¹, R², R³ and R⁴ are independently selected from the group of H, alkyl, alkylaryl, aryl and/or heteroaryl, and
   X is selected from halogen, such as chloro, bromo or fluoro, piperazinyl, which may be substituted or unsubstituted, preferably 4-methyl piperazinyl,
   R' is selected from H, formyl, COOAlkyl,
   with ammonium hydroxide of formula III

   R¹R²R³R⁴NOH (III)

   wherein R¹, R², R³ and R⁴ are as specified above,
b) reacting the obtained intermediate with formic acid and formaldehyde in the same reactor, and optionally,
c) in case X is halogen it is substituted by corresponding piperazine by conventional means to obtain after recovery compound of formula IV.

Preferably the reaction is carried out by reacting compound of formula II in which R is H, methyl, ethyl or R¹R²R³R⁴N cation, X is 4-methyl piperazinyl; R' is H and R¹, R², R³, R⁴ are the same and represent methyl or ethyl group.

Step a) is carried out under the same reaction conditions as the process for preparation compound of formula (I) according to first aspect of the present invention. Preferably the reaction is carried out in the neat conditions or in the absence of solvents. The reaction may take place at temperature between 60°C to 120°C, the reaction time required is from 1 to 20 hours. Preferably reaction is conducted for 4-8 hours at temperature from 80°C to 110°C. The ratio of compound of formula II to compound of formula III is from 1:1 to 1:10, preferably 1:3 to 1:7, most preferably from 1:4 to 1:6.

Step b) is carried out directly from the reaction mixture obtained after step a), without recovery of the product, by addition of formic acid and aqueous solution of formaldehyde. The molar ratio of compound of formula II : HCOOH : HCHO is preferably 1:5-30:1-6, more preferably 1:20-25:2-3. The reaction may take place at temperature between room temperature and 100°C, preferably at temperatures from 40°-80°C. The reaction time required is usually from 0.1 hours to 3 hours, preferably 0.25 to 0.5 hours. The compound thus prepared may be recovered from reaction mixture by conventional means, as free base or in the form of salt with acid or base. The compound may be recovered by crystallisation, precipitation or extraction.

The substitution according to optional step c) can be carried out according to processes known in the art. This reaction is preferably performed at reflux temperature in an organic solvent, such as toluene, in the presence of a base, such as a lower trialkylamine, e.g. triethylamine, or an alkali metal carbonate, e.g. sodium carbonate. Alternatively the reaction can be performed in a basic organic solvent such as pyridine, 1,4-diazabicyclo[2.2.2]-octane (DABCO), or triethylamine. The reaction can also be performed in neat piperazine, preferably in neat NMP. It was found that the rate of reaction depends on the total amount of amine (e.g. NMP) in the reaction mixture. In order to keep the amount of the nucleophilic piperazine as low as possible, another amine species can be added, preferably pyridine or a tertiary amine such as triethylamine, butylimidazole, or DABCO. The addition of at least equimolar amounts of these additional less nucleophilic amines is advantageous.

The product is recovered as a free base or in the form of salt by addition of water or organic solvent or mixtures thereof. In a preferred embodiment water and/or organic solvents are selected from C₁ - C₅ alcohol, acetonitrile or tetrahydrofuran, methyl tetrahydrofuran, acetic acid, formic acid, acetone and/or ethers are used. More preferably water and/or alcohols selected from methanol, ethanol and isopropanol; and ethers selected from tetrahydrofuran, methyl tetrahydrofuran, methyl t-butyl ether, diisopropyl ether are used.

Most preferably the product is recovered by addition of water affording precipitation of the product whereby byproducts remain dissolved in aqueous phase.

The obtained product may be further purified by conventional means such as crystallisation, maceration, precipitation and other conventional means. The product can be crystallised from alcohols such as methanol, ethanol, i-propanol, n-propanol; water; ethers, such as diethylether, toluene and a mixtures thereof. The product can be crystallised according to a processes disclosed in EP 259804, EP 680482, IPCOM 000186605D. The obtained crystal form of marbofloxacin can be the same as disclosed in IPCOM 000173285D.

The starting compounds of formula II can be prepared by any known process such as disclosed in EP 155587, EP 530360, US 4604401, RD 291097, EP 680482.

According to a particularly preferred embodiment of the present invention the compound which is reacted with ammonium hydroxide (III) to give compound (I) is a compound of formula (II) wherein both R and R' are hydrogen and X is 4-methyl piperazinyl (formula IIa). Compound (IIa) can be obtained according to EP 0 155 587 B1.

Compounds according to formula (II) which have a different structure than (IIa) are preferably first converted into compound (IIa). A preferred starting material for preparing compound (IIa) is a compound according to formula (II) wherein R is C₁ to C₆ alkyl, preferably ethyl, R' is formyl and X is F (formula IIb). Compound IIb can also be obtained according to EP 0 155 587 B1. The above reactions are summarised in Reaction Scheme 4.

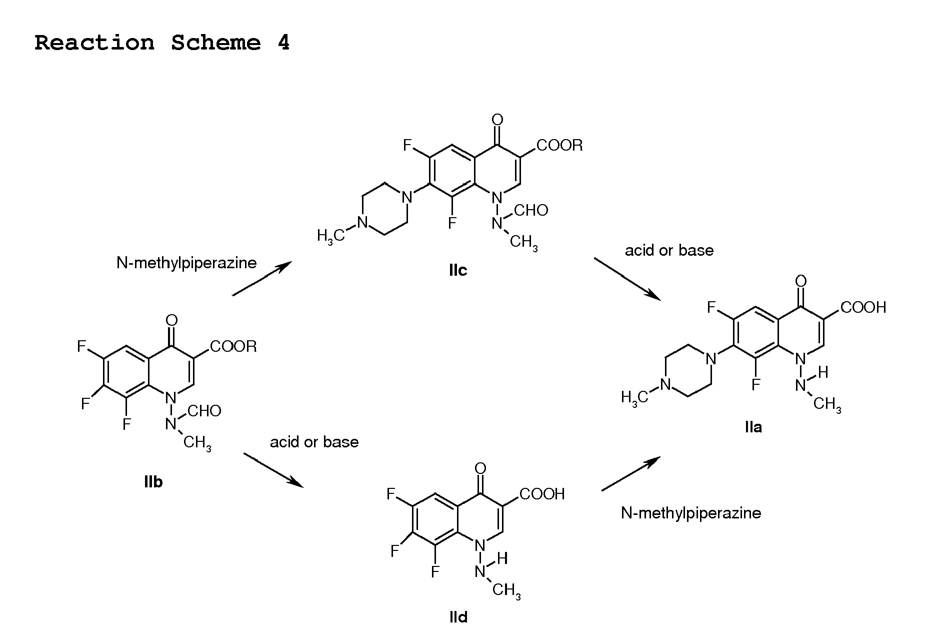

Compound (IIb) is reacted with N-methylpiperazine to yield the corresponding substitution product wherein X is 4-methyl piperazinyl (compound IIc). The substitution reaction can be performed according to EP 0 155 587 B1 or EP 0 680 482 B1. This reaction is preferably performed at reflux temperature in an organic solvent, such as toluene, in the presence of a base, such as a lower trialkylamine, e.g. triethylamine, or an alkali metal carbonate, e.g. sodium carbonate. Alternatively the reaction can be performed in a basic organic solvent such as pyridine, 1,4-diazabicyclo[2.2.2]octane (DABCO), or triethylamine. Since basic conditions promote the hydrolysis of the ester group and the formyl group often a mixture of (IIc) and (IIa) is obtained.

Compound (IIc) is then hydrolysed to give compound (IIa). Hydrolysis is achieved with a suitable base or, preferably with diluted acid, more preferably with aqueous HCl or H₂SO₄, such as 10% H₂SO₄. Since compound (IIc) is readily soluble in water, compound (IIc) is easily converted to (IIa). Compound (IIb) can be converted into (IIa) in a one-pot reaction without isolating (IIc).

According to an alternative process compound (IIb) is first hydrolysed to give compound (IId) followed by the substitution of the fluorine atom in position 7 to give compound (IIa). Hydrolysis of (IIb) can be achieved with diluted acid, preferably aqueous HCl or H₂SO₄, e.g. 10% H₂SO₄, or with a base such as a lower trialkylamine, e.g. triethylamine. An exemplary reaction medium for hydrolysis is a mixture of triethylamine (preferably about 2 to 4 vol.-%) in a solvent such as ethanol comprising a sufficient amount (about preferably 0.5 - 1 vol.-%) of water.

Compound (IIb) is hydrophobic and it is only poorly wetted by aqueous media. In order to facilitate mixing of compound (IIb) with aqueous media it is therefore preferably firstly mixed with a small amount of a polar solvent, preferably EtOH, MeCN or AcOH, before the hydrolysis reaction. These solvents form a film around the crystals of (IIb) and improve the wetting of this compound by aqueous solvents. In this way the hydrolysis reaction is significantly accelerated. Compound (IIb) can be treated with the polar solvent by simply mixing the solvent with compound (IIb), preferably in a ratio of at least 0.1 ml of solvent per mmol of compound (IIb).

For the substitution reaction the same conditions which are defined above can be used. Nucleophilic substitution of (IId) with a piperazine such as N-methylpiperazine (NMP) occurs regioselectively. (IIa) is the major reaction product. The reaction can be performed in neat piperazine, preferably in neat NMP, or is a solvent such as ethanol, water or a mixture thereof, preferably ethanol. It was found that the rate of reaction depends on the total amount of amine (e.g. NMP) in the reaction mixture. In order to keep the amount of the nucleophilic piperazine as low as possible, another amine species can be added, preferably pyridine or a tertiary amine such as triethylamine, butylimidazole, or DABCO. The addition of at least equimolar amounts of these additional less nucleophilic amines is advantageous. It is assumed that these amines scavenge HF that is generated during the reaction. HF can protonate the piperazine and thus reduces the nucleophilicity thereof. The addition of such amines does not influence the substitution reaction.

According to a preferred embodiment piperazine, in particular NMP, is used in an amount of 1-5 equivalents, more preferably 1-2 equivalents and most preferably 1.2 equivalents. The optional additional amine such as Et3N is preferably used in the same amount as the piperazine. Solvents such as EtOH can be used in any appropriate amount, e.g in an amount of 0-100mL/mmol, preferably 0.5-10mL/mmol or more preferably ImL/mmol of the starting material.

Compound (IIa) is then reacted with ammonium hydroxide (III), preferably with tetramethyl ammonium hydroxide, to give the corresponding compound of formula (I) which is then converted to marbofloxacin (IV) as described above. Thus, a preferred process of the present invention starts with compound (IIb) and proceeds via (IIc), (IIa), (I) to marbofloxacin. Alternatively, the process starts with compound (IIb) and proceeds via (IId), (IIa), (I) to marbofloxacin.

Dimeric impurities can be formed for instance by the reactions summarized in the reaction schemes 5 and 6. The reason is that N-methyl piperazine contains some amount of piperazine as an impurity (or can be converted to piperazine during the reaction), and reaction could proceed on both NH sites, thereby forming dimeric impurity.

Dimeric impurities have been associated with the problem of precipitation when preparing solutions such as injection or infusion solutions. It has been found by the inventors that the formation of precipitation from solution can be avoided and that the filterability of fluoroquinolone solutions can be improved by reducing the content of dimeric impurities to less than 0.01% (HPLC area), preferably less than 0.005% (HPLC area), more preferably to 0.003% (HPLC area).

The product purity is assessed by high pressure liquid chromatography (HPLC) with a pentafluorophenyl propyl (PFP) column (type Luna® PFP, 150 x 4.6mm, 3µm, Phenomenex, USA); detector: UV315 nm; flow rate: 0.8 ml/min; injection volume: 5 µl; mobile phase: A: 0.02M NaH₂PO₄xH₂O+0,1% TEA, pH2.5; B: acetonitrile : methanol = 5:95 (v/v) ; gradient: 0'=10B, 25'=100B, 30'= 100B, 32'=10B. This HPLC method is generally applicable for the 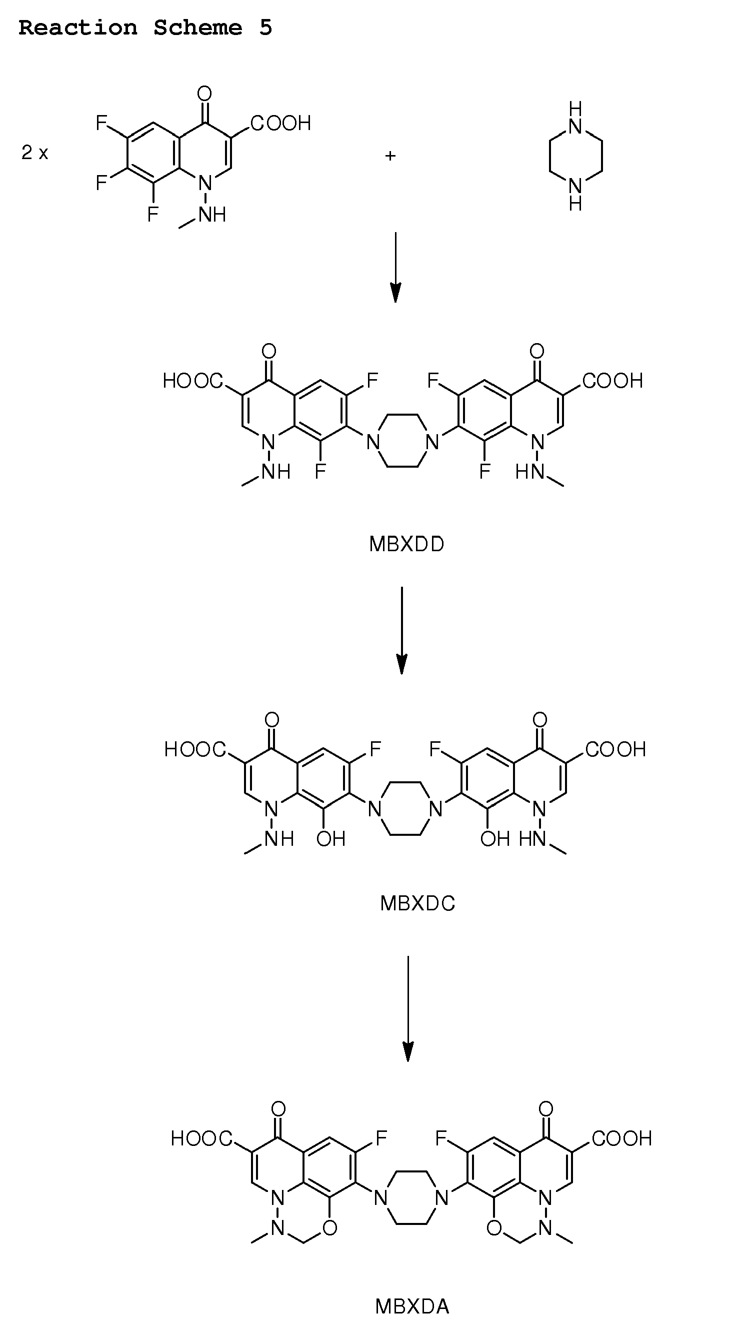 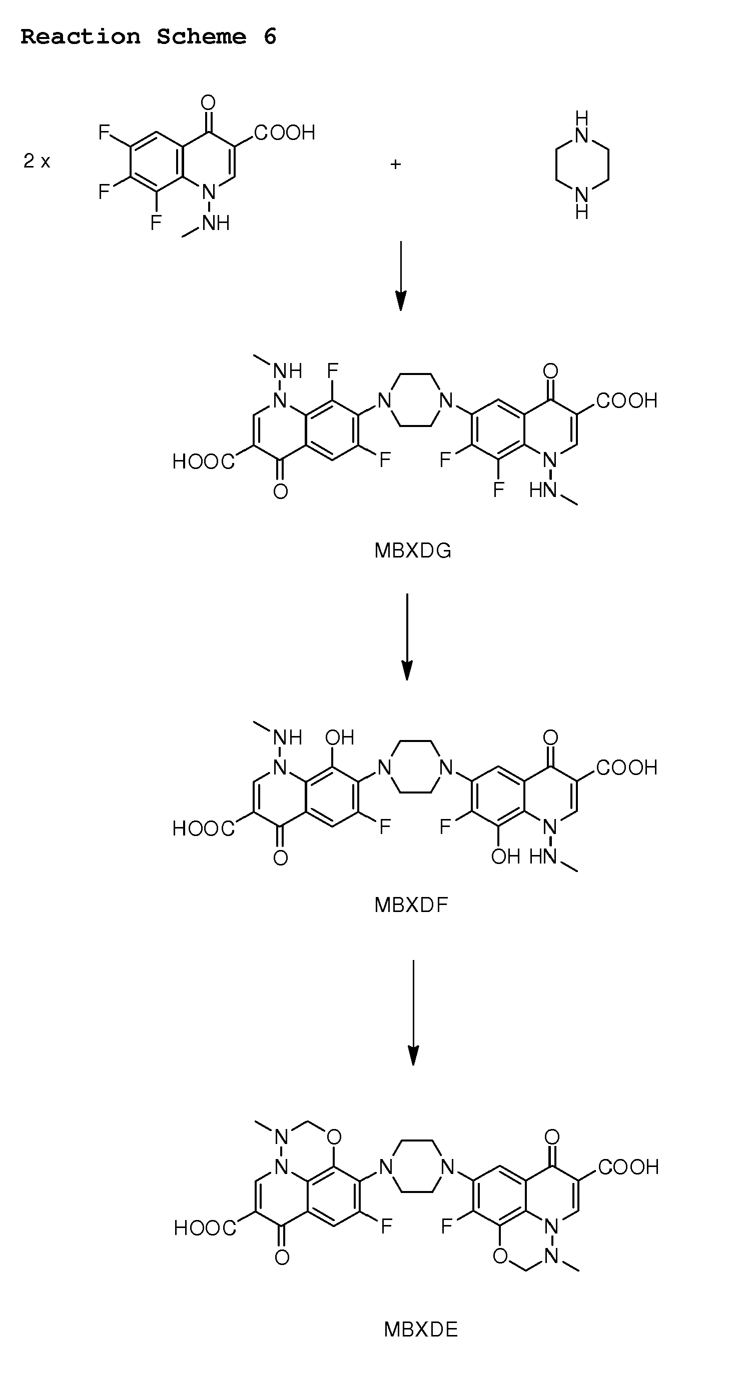 analysis of marbofloxacin, marbofloxacin derivatives, intermediates and precursors thereof containing a piperazine group, in particular those piperazine group containing compounds described herein. It was found that under the above conditions the retention time of dimeric impurities is at least two time higher than retention time of marbofloxacin.

The present invention also concerns a process for purification of marbofloxacin, marbofloxacin derivatives, intermediates and precursors thereof comprising a piperazine group. The purification method of the present invention comprises the following steps:
a') dissolving marbofloxacin or a marbofloxacin derivative, intermediate or precursor thereof comprising a piperazine group in water in the presence of an acid,
b') optionally adding activated carbon to the mixture of step (a'),
c') filtering the mixture of step (a') or (b') through filter, preferably an activated charcoal filter,
d') precipitating the product by addition basic reagent.

The marbofloxacin or piperazine group containing compound used in step (a') is preferably a compound synthesized according to the present invention. Thus, according to a third embodiment the present invention concerns a process which comprises the synthesis steps of the first embodiment or the second embodiment in combination with purification steps (a') to (d').

The solution obtained in step (a') or (b') is stirred for 0.5 to 24 hours, preferably 2-12 hours at temperatures from room temperature to 60°C.

The pH of the mixture of step (a') is preferably adjusted to a value within the range of 2,0 0 to 6,0, preferably in the range of 5.0 to 5.3.

The acid used in step (a') is preferably selected from organic acids, in particular mono-carboxylic acids and poly-carboxylic acids having from 2 to 25, preferably from 2 to 10, carbon atoms; monocyclic and polycyclic aryl acids such as benzoic acid. Preferred examples of these acids are acetic acid, formic acid, propanoic acid, methanesulfonic acid, malonic acid, succinic acid, fumaric acid, maleic acid, adipic acid, lactic acid, levulinic acid, sorbic acid and fruit acids such as tartaric acid, malic acid, ascorbic acid or citric acid. Particularly preferred organic acids are acetic acid, lactic acid, and gluconic acid. Alternatively, inorganic acids such as HCl can be used in step (a).

The pH of the mixture of step (b') is preferably adjusted to a value within the range of 6.4 to 8.0 preferably 6.8 to 7.4.

The basic reagent of step (d') is preferably an alkali metal hydroxide, such as sodium or potassium hydroxide, earth alkali metal hydroxides, ammonia or an amine, preferably an alkali metal hydroxide.

The purification method of the present invention is particularly suitable for removing impurities which tend to cause precipitations during manufacturing and storage of parenteral solutions, in particular dimeric impurities, most preferably the dimeric impurities shown in reaction schemes 5 and 6. Dimeric impurities can be removed at any stage of the synthesis where the piperazine group is already present in the molecule. Purification be performed either prior to substitution of the fluorine atom in position 8 by a hydroxyl group, prior to the cyclization reaction and/or at the stage of final product.

The purified marbofloxacin of the present invention has a purity of at least 99.5%, preferably 99.7%, more preferably 99.8%, most preferably 99.9% as determined by HPLC. In particular, the marbofloxacin of the present invention comprises less than 0.01% (HPLC area), preferably less than 0.005% (HPLC area), more preferably less than 0.003% (HPLC area) of dimeric impurities.

The dimeric impurities shown in reactions schemes 5 and 6 are useful as reference standards and are also subject of the present invention.

The compounds of formula (IV) prepared according to a process by the present invention can be used for the preparation of a pharmaceutical composition. Suitable pharmaceutical compositions comprise oral and parenteral compositions such as disclosed in WO 02/058669, WO 2008/030469, WO 2007/085760, WO 2007/101560, DE 10 2006 010 643, IPCOM000186605D. The pharmaceutical composition of the present invention are preferably filled into a packaging which complies with European Pharmacopeia standards.
Figure 1 shows an HPLC chromatogram of marbofloxacin prior to purification according to the method of the present invention, and
Figure 2 shows an HPLC chromatogram of marbofloxacin after to purification according to the method of the present invention, and

The following examples illustrate the invention without limiting it thereto.

### Examples

A high resolution HPLC method is used to determine an amount and purity compounds of formula I, II and IV. The tests are carried out in X-Bridge C18, 150 x 4.6mm, 3.5µm column. The mobile phase is gradient of A) 5mM NH₄COOCH₃ pH=7.0 B) acetonitrile. Gradient: 0'=10%B, 10'=20%B, 25'-30'=90%B, 32'=10%B.

The chromatograph is equipped with a UV detector set at 250 nm and 315nm, the flow rate is 1.0 ml per minute at 30°C.

### Example 1

### a) 6,8-difluoro-1-(methylamino)-7-(4-methylpiperazin-1-yl)-4-oxo-1,4-dihydroquinoline-3-carboxylic acid and 6,8-difluoro-1-(methylamino)-7-(4-methylpiperazin-1-yl)-4-oxo-1,4-dihydroquinoline-3-carboxylic acid sodium salt

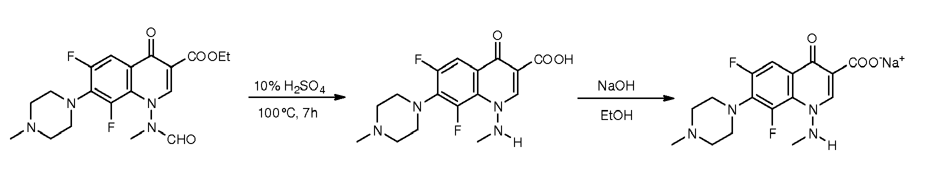

4.137g of Ethyl 6,8-difluoro-1-(N-methylformamido)-7-(4-methylpiperazin-1-yl)-4-oxo-1,4-dihydroquinoline-3-carboxylate (10.14mmol) was put into 40mL of 10% H₂SO₄ and stirred at 100°C for 7 hours. Reaction mixture was cooled and crystals were formed. Mixture was cooled to 4°C and filtered with suction. Filter cake was washed with a mixture of H₂O/EtOH/THF (1/1/5) and dried. 3.260g of 6,8-difluoro-1-(methylamino)-7-(4-methylpiperazin-1-yl)-4-oxo-1,4-dihydroquinoline-3-carboxylic acid as yellow crystals were obtained (91%).

In case the sodium salt is desired the product obtained in previous step was put into 5mL of EtOH and 10mL of CH₂Cl₂ and 1.20g of NaOH dissolved in 2mL of water was added. Solution was stirred at room temperature. for 1h, dried with Na₂SO₄ and evaporated. 2.90g of pure title product was isolated (yellow powder, 7.71mmol, 76%).

### b) 6,8-difluoro-1-(methylamino)-7-(4-methylpiperazin-1-yl)-4-oxo-1,4-dihydroquinoline-3-carboxylic acid

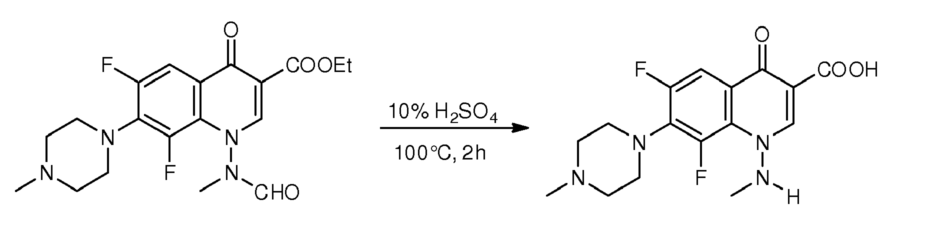

400mg of Ethyl 6,8-difluoro-1-(N-methylformamido)-7-(4-methylpiperazin-1-yl)-4-oxo-1,4-dihydroquinoline-3-carboxylate (0, 979mmol) was put into 2mL of 10% H₂SO₄ and stirred at 100°C for 2 hours. Reaction mixture was cooled and crystals were formed. To this mixture 1,7mL of 25% aq. NH₃ was slowly added. At first very dense suspension was formed that dissolves with further addition of ammonia solution. At the end clear solution formed with pH of 9. Ammonium sulphate was precipitated by the addition of 10mL of EtOH , filtered off and washed with 5mL of H₂O/EtOH (1/2). Mother liquor was dried on the rotary evaporator and 10 mL of EtOH/H₂O mixture (7/3) was added to precipitate residual inorganic salt, which was again filtered off. Remaining yellow solution was dried on a rotary evaporator to obtain 321mg of yellow powder (0.912 mmol, 93%).

### Example 2

### 6-fluoro-8-hydroxy-1-(methylamino)-7-(4-methylpiperazin-1-yl)-4-oxo-1,4-dihydroquinoline-3-carboxylic acid

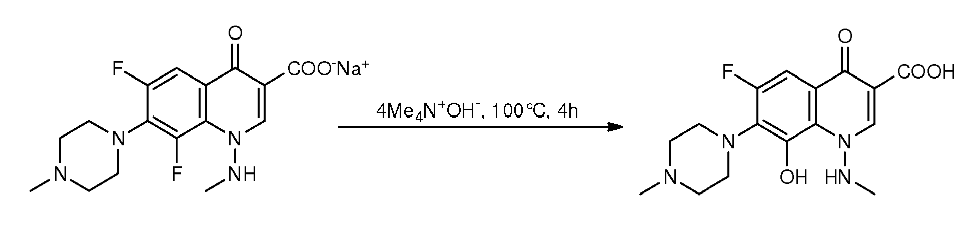

178 mg of 6,8-difluoro-1-(methylamino)-7-(4-methyl-piperazin-1-yl)-4-oxo-1,4-dihydroquinoline-3-carboxylic acid sodium salt (0.470mmol) was mixed with 360 mg of Me₄NOH.5H₂O (2.00 mmol) and stirred at 100°C for 4 hours. Ammonium salt melts and dark brown oil is formed during the reaction. Reaction mixture was cooled to room temperature and 0.10mL of HCOOH was added to neutralize hydroxide. 5mL of EtOH is added to precipitate the product, which was filtered with suction and filter cake was washed with 2mL of cold EtOH. 90mg of the product was obtained.

### Example 3

### 9-fluoro-3-methyl-10-(4-methylpiperazin-1-yl)-7-oxo-3,7-dihydro-2H-[1,3,4]oxadiazino[6,5,4-ij]quinoline-6-carboxylic acid formate salt

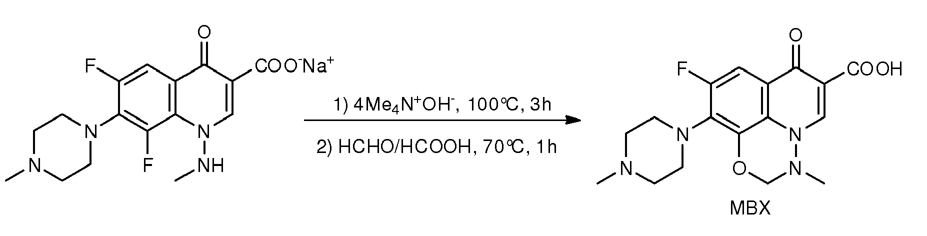

180 mg of 6,8-difluoro-1-(methylamino)-7-(4-methylpiperazin-1-yl)-4-oxo-1,4-dihydroquinoline-3-carboxylic acid sodium salt (0.481mmol) was mixed with 360 mg of Me₄NOH.5H₂O (2.00 mmol) and stirred at 100°C for 3 hours. Ammonium salt melts and dark brown oil is formed during the reaction. Reaction mixture was cooled to room temperature and 1 mL of HCOOH was added followed by addition of 0.4 mL of 37% aq. solution of HCHO and stirred at 70°C for additional hour. Reaction mixture was cooled to room temperature and 5mL of EtOH was added to precipitate the product, which was filtered with suction and filter cake was washed with 2mL of cold EtOH. 111 mg of grey powder was obtained.

### Example 4

### 9-fluoro-3-methyl-10-(4-methylpiperazin-1-yl)-7-oxo-3,7-dihydro-2H-[1,3,4]oxadiazino[6,5,4-ij]quinoline-6-carboxylic acid formate salt

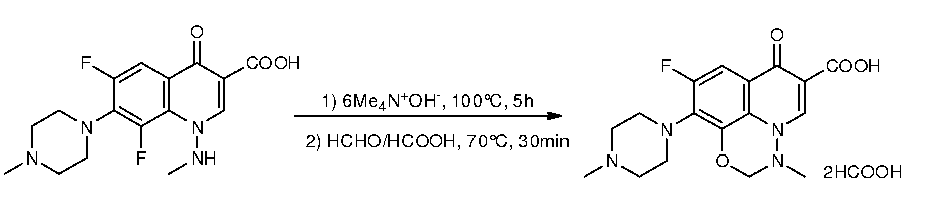

1.14g of 6,8-difluoro-1-(methylamino)-7-(4-methyl-piperazin-1-yl)-4-oxo-1,4-dihydroquinoline-3-carboxylic acid (3.00mmol) was mixed with 3.06g of Me₄NOH.5H₂O (16.96mmol) and stirred at 100°C for 5 hours. Ammonium salt melts and dark brown oil is formed during the reaction. Reaction mixture was cooled to room temperature and 1.44 mL of HCOOH (85% aq. sol) was added followed by addition of 0.5 mL of 37o aq. solution of HCHO and the flask was cooled on the water bath at 22°C. Another 1.44mL of 85% HCOOH was added and the reaction mixture was warmed to 70°C for 30min and after cooling 20mL of EtOH was added to the reaction mixture and left in a refrigerator for 16h. Precipitate was filtered under reduced pressure and washed with cold ethanol (10mL). After drying 1.23g of grayish powder was obtained (90%) .

### Example 5

### 9-fluoro-3-methyl-10-(4-methylpiperazin-1-yl)-7-oxo-3,7-dihydro-2H-[1,3,4]oxadiazino[6,5,4-ij]quinoline-6-carboxylic acid

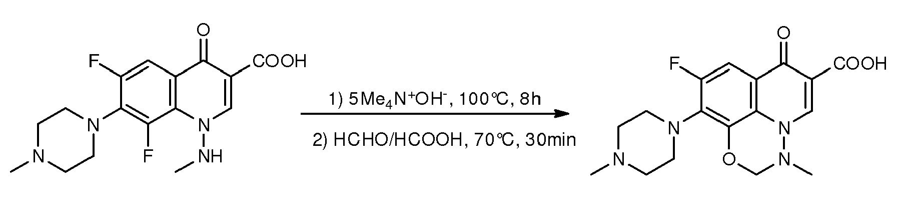

1.145g of 6,8-difluoro-1-(methylamino)-7-(4-methyl-piperazin-1-yl)-4-oxo-1,4-dihydroquinoline-3-carboxylic acid (3.01mmol) was mixed with 2.72g of Me₄NOH.5H₂O (15.00mmol) and stirred at 100°C for 8 hours. Ammonium salt melts and dark brown oil is formed during the reaction. Reaction mixture was cooled to room temperature and 3.0 mL of HCOOH was added followed by addition of 0.5 mL of 37% aq. solution of HCHO (6.0mmol) and the flask was cooled on the water bath at 22°C. Precipitate was immediately formed. The flask was warmed to 70°C, during which precipitate was dissolved. After stirring at 70°C for 30min (precipitate was formed again after 5min) reaction flask was cooled to room temperature and 20mL of EtOH was added to the reaction mixture and left in a refrigerator for 16h. Precipitate was filtered under reduced pressure and washed with cold ethanol (10mL). After drying 1.165g of grayish powder was obtained (85%), with a purity of 97.11% (HPLC).

Crude reaction product was mixed with 0.9mL of 25% NH3 aqueous solution and crystallized in a mixture of 26mL of EtOH and 14mL H₂O. 0.673g of powder was obtained (61%) with a purity of 98.75% (HPLC).

### Example 6

### 9-fluoro-3-methyl-10-(4-methylpiperazin-1-yl)-7-oxo-3,7-dihydro-2H-[1,3,4]oxadiazino[6,5,4-ij]quinoline-6-carboxylic acid

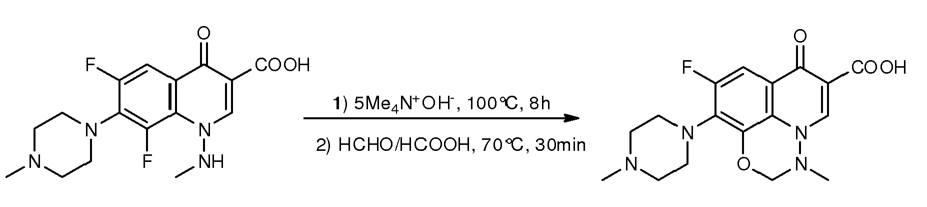

1.140g of 6,8-difluoro-1-(methylamino)-7-(4-methyl-piperazin-1-yl)-4-oxo-1,4-dihydroquinoline-3-carboxylic acid (3.00mmol) was mixed with 2.72g of Me₄NOH.5H₂O (15.01mmol) and stirred at 100°C for 8 hours. Ammonium salt melts and dark brown oil is formed during the reaction. Reaction mixture was cooled to room temperature and 3.0 mL of HCOOH was added followed by addition of 0.5 mL of 37% aq. solution of HCHO (6.0mmol) and the flask was cooled on the water bath at 22°C. Precipitate was immediately formed. The flask was warmed to 70°C, during which precipitate was dissolved and stirred for 30 min (after stirring for at 70°C for 5min precipitate formed again). Reaction flask was cooled to room temperature and 20mL of H₂O was added to the reaction mixture and left in a refrigerator for 16h. Precipitate was filtered under reduced pressure and washed with cold ethanol (10mL). After drying 1.022g of greyish powder was obtained (75%). with a purity of 97.11% (HPLC).

Crude reaction product was mixed with 0.9mL of 25% NH₃ aqueous solution and crystallised in a mixture of 20mL of EtOH and 6mL CHCl₃. 0.771g of yellow powder was obtained (71%) with a purity of 99.50% as determined by HPLC.

### Example 7

### 9-fluoro-3-methyl-10-(4-methylpiperazin-1-yl)-7-oxo-3,7-dihydro-2H-[1,3,4]oxadiazino[6,5,4-ij]quinoline-6-carboxylic acid

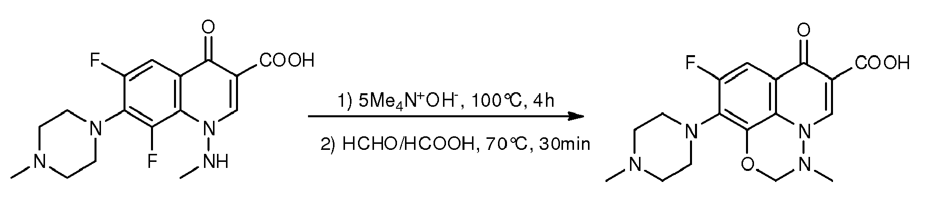

1.142 g of 6,8-difluoro-1-(methylamino)-7-(4-methyl-piperazin-1-yl)-4-oxo-1,4-dihydroquinoline-3-carboxylic acid (3.01mmol) was mixed with 3.26g of Me₄NOH.5H₂O (18.01mmol) and stirred at 100°C for 4 hours. Ammonium salt melts and dark brown oil is formed during the reaction. Reaction mixture was cooled to room temperature and 3.0 mL of HCOOH was added followed by addition of 0.5 mL of 37% aq. solution of HCHO (6.0mmol) and the flask was cooled on the water bath at 22°C. Precipitate was immediately formed. The flask was warmed to 70°C, during which precipitate was dissolved and stirred for 30 min (after stirring for at 70°C for 5min precipitate formed again). Reaction flask was cooled to room temperature and dried on the rotary evaporator. 20mL of H₂O was added to the reaction mixture and cooled in a refrigerator. Precipitate was filtered under reduced pressure. After drying 1.147g of white powder was obtained (84%).

Crude reaction product was mixed with 5mL of water and 2mL of 25% aqueous solution of NH₃ and clear solution was obtained. To this solution, 7mL of EtOH was added and dried under reduced pressure. Product was crystallized in a mixture of 15mL of EtOH and 10mL CHCl₃ to obtain 0.4321g of white powder (41%) with a purity of 98.63% as determined by HPLC

### Example 8

### 9-fluoro-3-methyl-10-(4-methylpiperazin-1-yl)-7-oxo-3,7-dihydro-2H-[1,3,4]oxadiazino[6,5,4-ij]quinoline-6-carboxylic acid

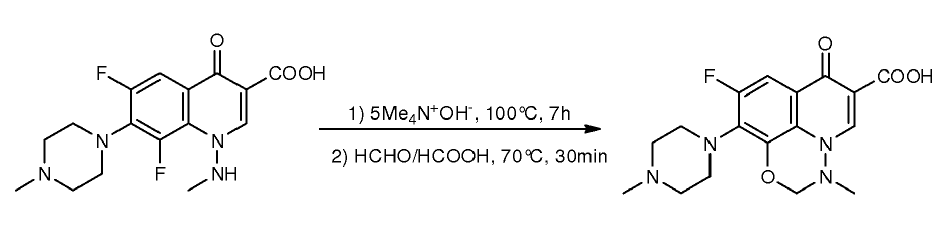

1.136 g of 6,8-difluoro-1-(methylamino)-7-(4-methyl-piperazin-1-yl)-4-oxo-1,4-dihydroquinoline-3-carboxylic acid (2.98mmol) was mixed with 2.73g of Me₄NOH.5H₂O (15.00mmol) and stirred at 100°C for 7 hours. Ammonium salt melts and dark brown oil is formed during the reaction. Reaction mixture was cooled to room temperature and 3.0 mL of HCOOH was added followed by addition of 0.5 mL of 37% aq. solution of HCHO (6.0mmol) and the flask was cooled on the water bath at 22°C. Precipitate was immediately formed. The flask was warmed to 70°C, during which precipitate was dissolved and stirred for 30 min (after stirring for at 70°C for 5min precipitate formed again). Reaction flask was cooled to room temperature and dried on the rotary evaporator. 20mL of H₂O was added to the reaction mixture and cooled in a refrigerator. Precipitate was filtered under reduced pressure. After drying 1.039g of grey powder was obtained (77%).

Crude reaction product was neutralized with 2mL of 25% aqueous solution of NH₃ and clear solution was diluted with 15mL of EtOH and 9mL of H₂O. Solution was partially dried under reduced pressure until the formation of precipitate. At this point mixture was cooled in a refrigerator and precipitate was isolated by filtration under reduced pressure to obtain 0.675g of powder (65%) with a purity of 98.84% as determined by HPLC.

### Example 9

### 9-fluoro-3-methyl-10-(4-methylpiperazin-1-yl)-7-oxo-3,7-dihydro-2H-[1,3,4]oxadiazino[6,5,4-ij]quinoline-6-carboxylic acid

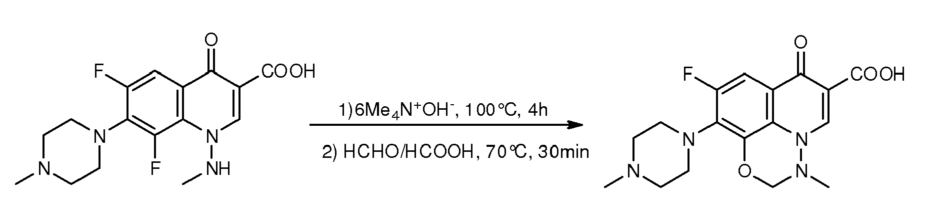

1.140g of 6,8-difluoro-1-(methylamino)-7-(4-methyl-piperazin-1-yl)-4-oxo-1,4-dihydroquinoline-3-carboxylic acid (3.01mmol) was mixed with 3.30g of Me₄NOH.5H₂O (18.20mmol) and stirred at 100°C for 4 hours. Ammonium salt melts and dark brown oil is formed during the reaction. Reaction mixture was cooled to room temperature and 3.0 mL of HCOOH was added followed by addition of 0.5 mL of 37% aq. solution of HCHO (6.0mmol) and the flask was cooled on the water bath at 22°C. Precipitate was immediately formed. The flask was warmed to 70°C, during which precipitate was dissolved and stirred for 30 min (after stirring for at 70°C for 5min precipitate formed again). Reaction flask was cooled to room temperature and dried on the rotary evaporator. 20mL of H₂O was added to the reaction mixture and cooled in a refrigerator. Precipitate was filtered under reduced pressure to obtain 0.847g of solid, while mother liquid was diluted with EtOH and concentrated under reduced pressure until precipitate forms, which was filtered again to obtain additional 0.208g of solid. The yield of combined solid material is 1.055g, 77%. Crude reaction product (formate salt) was crystallized in H₂O/EtOH (25mL/10mL) to obtain 0.722g (53%) of yellow powder. Formate salt was put in 20mL of EtOH/CH₂Cl₂ mixture (1/1) and 0.5mL of 25%aq. NH₃ was added to obtain clear solution. Solution was dried with Na₂SO₄ and solvent evaporated under reduced pressure to obtain 0.580g of yellow powder (53%).

### Example 10

### 9-fluoro-3-methyl-10-(4-methylpiperazin-1-yl)-7-oxo-3,7-dihydro-2H-[1,3,4]oxadiazino[6,5,4-ij]quinoline-6-carboxylic acid

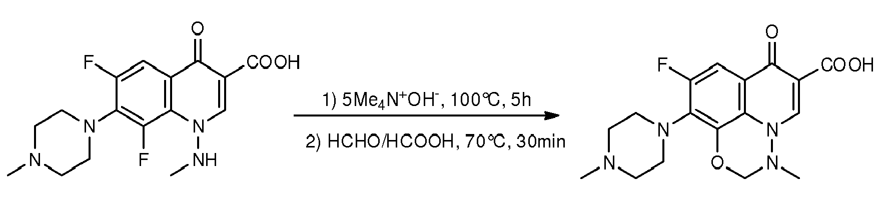

100 mL reactor with a rotary stirrer was charged with 10,16g of 6,8-difluoro-1-(methylamino)-7-(4-methyl-piperazin-1-yl)-4-oxo-1,4-dihydroquinoline-3-carboxylic acid (28,83mmol) and 26,50g of Me₄NOH˙5H₂O (146,25mmol) that was previously mixed together. Temperature of the heating jacket was set to 100°C and stirring to 100s⁻¹, while water was allowed to evaporate out of the reactor during the reaction. Reaction was stirred at specified temperature for 5 hours and homogenous dark brown oil was obtained. Temperature of reactor was cooled to 20°C, 30mL of HCOOH was added and stirred well so that all oil is transformed into brown suspension. 4,5mL of 37% aq. HCHO was added drop-wise and heated at 70°C for 30min. Reaction mixture was cooled to 20°C and 20mL of water added to precipitate the product in the form of formate complex. Suspension was cooled to 0°C and filtered under reduced pressure and washed the filter cake with additional 10mL of cold water to obtain 8,38g of white powder. Mother liquor was partially evaporated under reduced pressure and when solid started to precipitate it was filtered again to obtain additional 0.80g of powder. 50mL of EtOH was added into the mother liquor to precipitate the product and after filtration at reduced pressure further 0.80g of white powder was obtained. Product was collected and 9,98g of white powder was suspended in a mixture of 50mL of EtOH and 50mL of CH₂Cl₂. Into the suspension 25% aq. NH₃ was added to neutralize the formate complex and after addition of 12mL of NH₃ all product was dissolved and small amount of solid material is formed. 5g of anhydrous Na₂SO₄ was added to dry the organic solution and it was filtered off and solvent evaporated under reduced pressure. 8.99g of slightly yellow powder was obtained in 86% yield.

### Example 11

### Crystallization from ethanol/toluene/water 2:1:1

8.4g of crude marbofloxacin was suspended in a mixture of 83 ml of ethanol, 41ml of toluene and 41 ml of water and heated to reflux. From the clear yellow solution formed 83 ml of solvent mixture was distilled off, whereby the temperature rose from 74 to about 79°C, and a yellow precipitate was formed. The suspension was cooled to 20° - 25°C, stirred for 1 hour, filtered, and the filter cake was washed with 3 portions of 6 ml of ethanol to yield after drying in vacuum dryer the product in more than 95% yield.

### Example 12

### Crystallization of marbofloxacin starting from marbofloxacin formate

26g of marbofloxacin formate was suspended in a mixture of 65ml of ethanol and 27ml of water. Under stirring a solution of 25% ammonia in ethanol (20ml 25%NH3/10ml EtOH) is slowly (about 30 minutes) added by drops until the substance is dissolved and pH value of 7-9 is reached. The reaction was stirred for about 15 minutes and filtered. The filtrate was evaporated at 110°C until about 60ml of the solvent was distilled off and marbofloxacin started to precipitate. After distillation the suspension was cooled and stirred for 0.5 to 1 hour at 0-5°C, filtered, to yield after drying at 40°C/50mbar for 3 to 5 hours the product in 100%yield.

### Example 13

### Crystallization from ethanol

1g of marbofloxacin was dissolved under heating to reflux in 160ml of ethanol, after filtration, the solution is cooled and the crystallized product is recovered in more than 90% yield.

### Example 14

### 6,7,8-Trifluoro-1-methylamino-1,4-dihydro-4-oxo-3-quinoline-carboxylic acid

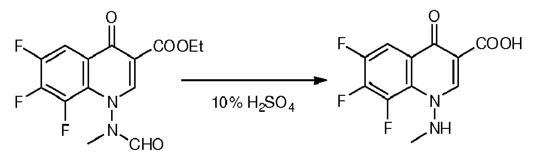

10mmol of 6,7,8-Trifluoro-1-(N-methylformamido)-1,4-dihydro-4-oxo-3-quinoline-carboxylic acid ethyl ester was put in the round bottomed flask. 20mL of 10% H₂SO₄ was added and stirred with the temperature of the sand bath of 100°C for the time periods specified in the following table. Reaction mixture was cooled down to 4°C, filtered and the cake washed with water and the conversion an yield were determined.

The experiment was repeated but starting compound was mixed with 1.0mL of solvent (EtOH, AcOH or MeCN as specified in the following table) before adding the 10% H₂SO₄.

The starting compound is insoluble in aqueous phase. By mixing the starting compound with a small amount of polar solvent (EtOH, MeCN, AcOH) a film is formed around the crystals which improves wetting of the crystals with the aqueous acid. Without addition of polar solvent prior to adding the aqueous acid solution wetting of the crystals is impaired and the reaction is slower.

| **Exp.** | **Reaction time (solvent)** | **Conversion (yield)** |
|---|---|---|
| 14.01 | 6h | 65% |
| 14.02 | 7h | 60% |
| 14.03 | 24h | 100% |
| 14.04 | 24h | 100% (94%) |
| 14.05 | 6h (0.1mL AcOH per mmol) | 91% |
| 14.06 | 6h (0.1mL EtOH per mmol) | 89% |
| 14.07 | 21h (0.1mL MeCN per mmol) | 100% (97%) |
| 14.08 | 21h (0.1mL MeCN per mmol) | 100% (96%) |

### Example 15

### 6,7,8-Trifluoro-1-methylamino-1,4-dihydro-4-oxo-3-quinoline-carboxylic acid

3.30g of 6,7,8-Trifluoro-1-(N-methylformamido)-1,4-dihydro-4-oxo-3-quinoline-carboxylic acid ethyl ester (10.054 mmol) was put into the round-bottomed flask equipped with the magnetic stirrer. 1mL of MeCN was added and stirred for a minute. 20mL of 10% H₂SO₄ was added and stirred. The flask was put into the sand bath (T = 100°C) and stirred for 21h. Suspension was cooled down to 4°C and filtered under suction. Yellow powder was washed twice with cold water and dried. 2.646g of yellow powder was obtained (9.721 mmol, 96.7%) and identified by NMR spectroscopy to be title compound.

### Example 16

### 6,7,8-Trifluoro-1-methylamino-1,4-dihydro-4-oxo-3-quinoline-carboxylic acid

6,7,8-Trifluoro-1-(N-methylformamido)-1,4-dihydro-4-oxo-3-quinoline-carboxylic acid ethyl ester (6.868g, 20.92 mmol) was mixed with 1mL of EtOH (to decrease the hydrophobicity of the substrate). Next, 40mL of 10% aqueous H₂SO₄ solution was added and the mixture was stirred at the temperature of the bath of 100°C for 12h. A white suspension formed which was cooled to 0°C and filtered under reduced pressure. The white powder was washed with cold water and cold EtOH and dried. 5.135g of yellow powder was obtained and identified as title compound by ¹⁹F and ¹H NMR spectroscopy. The yield of hydrolysis was 90%.

### Example 17

### 6,8-Difluoro-1-(methylamino)-7-(4-methylpiperazin-1-yl)-4-oxo-1,4-quinoline-3-carboxylic acid

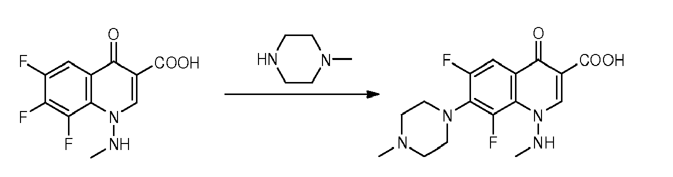

6,7,8-Trifluoro-1-methylamino-1,4-dihydro-4-oxo-3-quinoline-carboxylic acid (272mg, 1.0mmol, obtained as described in Example 16, and 400 mg of N-methylpiperazine (4.0mmol) were mixed with 1mL of EtOH and stirred under reflux temperature (jacket temperature Tj=100°C). After two hours of reaction clear solution formed, afterwards the product precipitated and a very dense suspension was formed. Reaction was stopped after three hours of stirring at Tj=100°C. A sample was put directly to the NMR analysis and only two signals were observed indicating reaction was quantitative. Crude reaction product was diluted with EtOH and neutralized by addition of aqueous solution of NH₃ until pH of 8 was reached. Suspension was cooled to 0°C and product isolated by filtration under reduced pressure, washed further with 10mL of cool EtOH and dried. 138mg (39%) of product was obtained.

### Example 18

### 6,8-Difluoro-1-(methylamino)-7-(4-methylpiperazin-1-yl)-4-oxo-1,4-quinoline-3-carboxylic acid

6,7,8-Trifluoro-1-methylamino-1,4-dihydro-4-oxo-3-quinoline-carboxylic acid (1.087g, 3.993mmol), 484mg of N-methylpiperazine (4.83mmol) and 484 mg of Et₃N (4.78mmol) were mixed with 8mL of EtOH and stirred under reflux temperature (Tj=100°C). After 19h of reflux yellow solution and white precipitate are formed in the reaction flask. Solvent was evaporated under reduced pressure and put directly to the NMR analysis. Crude reaction product was mixed with 20mL of EtOH and suspension cooled in the refrigerator. The product (white precipitate) was isolated by filtration under reduced pressure, washed further with 10mL of cool EtOH and dried. 1.178g of white powder was obtained (3.375 mmol, 800).

### Example 19

### 6,8-Difluoro-1-(methylamino)-7-(4-methylpiperazin-1-yl)-4-oxo-1,4-quinoline-3-carboxylic acid

In accordance with examples 17 and 18 additional experiments were carried out using different reaction conditions for the conversion of 6,7,8-trifluoro-1-methylamino-1,4-dihydro-4-oxo-3-quinoline-carboxylic acid into 6,8-difluoro-1-(methylamino)-7-(4-methylpiperazin-1-yl)-4-oxo-1,4-quinoline-3-carboxylic acid. The experiments were performed according to the following general procedure: 1.0mm of starting compound was put in the round bottomed flask and N-methylpiperazine (NMP), base and solvent were added according to the following table. Reaction mixture was stirred at the corresponding temperature. Solvent was evaporated and crude reaction mixture analyzed directly by NMR (¹H and ¹⁹F).

| **Exp.** | **NMP** (equivalents) | **base** (equivalents) | **solvent (1M)** | **reaction conditions** | **conversion (yield)** |
|---|---|---|---|---|---|
| **19.01** | 1.2 | / | / | 100°C, 16h | 54% |
| **19.02** | 4 | / | / | 80°C, 2h | 100% |
| **19.03** | 2.5(MBX61 Na⁺ salt) | / | / | 100°C, 3h | 100% |
| **19.04** | 4 | / | EtOH | reflux, 3h | 97% |
| **19.05** | 4 | / | EtOH | reflux, 6h | 100% |
| **19.06** | 3 | / | EtOH | reflux, 2h | 95% |
| **19.07** | 3 | / | EtOH | reflux, 2.5h | 96% |
| **19.08** | 3 | / | EtOH | reflux, 3h | 100% |
| **19.09** | 2 | / | EtOH | reflux, 3h | 94% (71%) |
| **19.10** | 2 | / | EtOH | reflux, 6h | 100% |
| **19.11** | 1.1 | / | EtOH | reflux, 6h | 23% |
| **19.12** | 1.1 | / | EtOH(0.25M) | reflux, 6h | 48% |
| **19.13** | 1.1 | / | EtOH(0.25M) | reflux, 16h | 84% |
| **19.14** | 1.2 | / | H₂O(30equiv.) | 100°C, 16h | 89% |
| **19.15** | 3 | / | H₂O(1M) | 100°C, 2h | 95% |
| **19.16** | 1.1 | 3 NaHCO₃ | EtOH | reflux, 6h | 26% |
| **19.17** | 1.1 | 3 NaHCO₃ | EtOH | reflux, 22h | 37% |
| **19.18** | 4 | 1 DABCO | / | 80°C, 2h | 100% |
| **19.19** | 3 | 4 DABCO | / | 80°C, 2h | 100% |
| **19.20** | 4 | 1 BIM | / | 100°C, 1h | 100% |
| **19.21** | 3 | 4 BIM | / | 90°C, 2h | 77% |
| **19.22** | 1.2 | 2 Py | / | 100°C, 16h | 98% |
| **19.23** | 1.2 | 2 Et₃N | / | 100°C, 16h | 77% |
| **19.24** | 1.1 | 3 Et₃N | EtOH | reflux, 6h | 92% |
| **19.25** | 1.1 | 2 Et₃N | EtOH | reflux, 6h | 92% |
| **19.26** | 1.1 | 2 Et₃N | EtOH | reflux, 22h | 99% |
| **19.27** | 1.2 | 1.1 Et₃N | EtOH (0.5M) | reflux, 20h | 99% |
| **19.28** | 1.2 | 1.2 Et₃N | EtOH (0.5M) | reflux, 19h | 96% (80%) |
| **19.29** | 1.2 | 1.2 Et₃N | EtOH(0.5M) | reflux, 8h | 91% |
| **19.30** | 1.2 | 1.2 Et₃N | EtOH (0.5M) | reflux, 22h | 100% |
| **19.31** | 1.2 | 1.5 Et₃N | EtOH (0.5M) | reflux, 8h | 88% |
| **19.32** | 1.2 | 1.5 Et₃N | EtOH (0.5M) | reflux, 22h | 100% |
| **19.33** | 1.2 | 2 Et₃N | EtOH (0.5M) | reflux, 8h | 91% |
| **19.34** | 1.2 | 2 Et₃N | EtOH (0.5M) | reflux, 22h | 100% |

| | | | | | |
|---|---|---|---|---|---|
| Py - pyridine; BIM - N-butylimidazole; DABCO - 1,4-Diazabicyclo[2.2.2]octan | | | | | |

The nucleophilic substitution in the 6,7,8-trifluoro-1-methylamino-1,4-dihydro-4-oxo-3-quinoline-carboxylic acid with N-methylpiperazine (NMP) occurred regioselectively. 6,8-Difluoro-1-(methylamino)-7-(4-methylpiperazin-1-yl)-4-oxo-1,4-quinoline-3-carboxylic acid was the major reaction product. Reaction was performed in neat NMP and in different solvents (ethanol, water). It was found that the rate of reaction depends on the total amount of amine (NMP) in the reaction mixture. The addition of at least equimolar amounts of the less nucleophilic amines, triethylamine, pyridine, butylimidazole (BIM), or DABCO proved to be advantageous for scavenging HF that is formed during the reaction. The addition of these tertiary amines did not influence the conversion whereas the addition of inorganic NaHCO₃ proved to be disadvantageous.

### Example 20

### 6,8-Difluoro-1-(N-methylformamido)-7-(4-methylpiperazin-1-yl)-4-oxo-1,4-quinoline-3-carboxylic acid ethyl ester

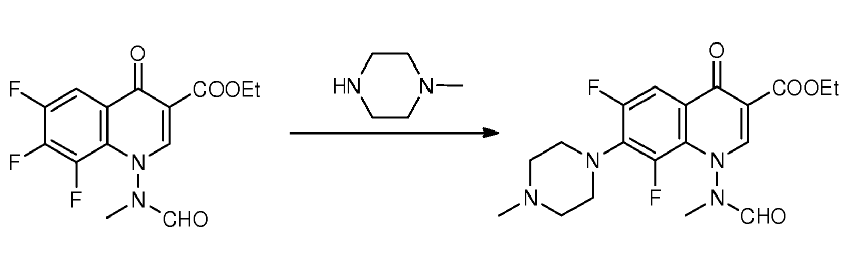

***Substitution:*** 6,7,8-Trifluoro-1-(N-methylformamido)-4-oxo-1,4-quinoline-3-carboxylic acid ethyl ester (1.0 mmol, 324mg) was mixed with 2 equivalents of N-methylpiperazine (220mg) and 400mg Et₃N stirred for three hours at 100°C. Reaction mixture liquefied in 10 minutes and solidified again within 30 minutes of the reaction (that is the reason for higher amount of TEA). After 3 hours of stirring was reaction mixture cooled to room temperature and analyzed by NMR spectroscopy.

**Substitution:** The above reaction was repeated but Et₃N was replaced by 1 equivalent of DABCO.

In both cases, substitution was quantitative and analysis of the crude reaction mixtures showed that there was some hydrolysis of the ethyl ester (EE) to the free carboxylic acid (CA) group resulting in a product mixture. The results are summarized in the following table. Ethyl ester is readily soluble in water.

| **Exp.** | **Reaction conditions** | **Conversion (yield)** |
|---|---|---|
| 20.01 | 2.5 NMP, 1 DABCO, 100°C, 3h | 100% (48% EE, 52% CA) |
| 20.02 | 2.5 NMP, 4 Et₃N, 100°C, 3h | 100% (58% EE, 42% CA) |

### Example 21

### 6,8-Difluoro-1-(methylamino)-7-(4-methylpiperazin-1-yl)-4-oxo-1,4-quinoline-3-carboxylic acid (one-pot reaction)

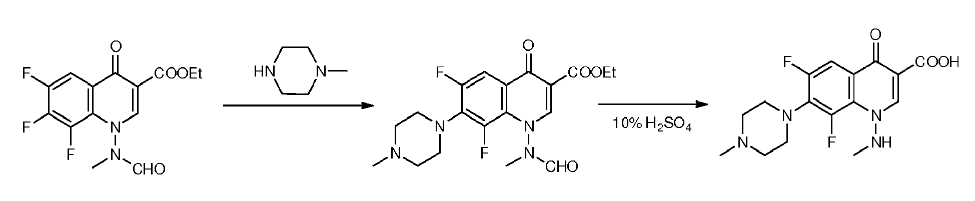

6,7,8-Trifluoro-1-(N-methylformamido)-4-oxo-1,4-quinoline-3-carboxylic acid ethyl ester (1.0 mmol, 324mg) was mixed with 2 equivalents of N-methylpiperazine (200mg) and stirred for one hour at 100°C. Reaction mixture liquefied in 10 minutes and solidified again within 30 minutes of reaction. After one hour of reaction the reaction mixture was cooled to room temperature and 10% aqueous H₂SO₄ (5mL) was added and stirred again at 100°C for two hours. Yellow solution was cooled to 0°C so that product precipitated. It was isolated by filtration under reduced pressure. Pure 6,8-difluoro-1-(methylamino)-7-(4-methylpiperazin-1-yl)-4-oxo-1,4-quinoline-3-carboxylic acid in the form of sulfate salt was obtained (as determined by NMR) as slightly yellow powder (279mg, 58%).

### Example 22

### Synthesis of 9-fluoro-3-methyl-10-(4-methylpiperazin-1-yl)-7-oxo-3, 7-dihydro-2H-[1,3,4]oxadiazino[6,5,4-ij]quinoline-6-carboxylic acid (Marbofloxacin, MBX)

13.5 g of 6,8-Difluoro-1-(methylamino)-7-(4-methyl-piperazin-1-yl)-4-oxo-1,4-dihydroquinoline-3-carboxylic acid hydrochloride and ca. 63 g of tetramethylammonium hydroxide water solution 25 % were charged into a reactor and slowly heated to 100°C. When this temperature was reached, water was removed by distillation at reduced pressure (between 0.8 to 0.3 bar) in such a manner that ca. 25 to 32 ml of water were removed in 3 hours. The reaction mixture was stirred for another 3 hours and after completion of the conversion, the reaction mixture was cooled to 0 - 10 °C and ca. 40.5 ml of formic acid were slowly added with violent agitation. The temperature was maintained below 20°C, preferable between 0 - 10°C. Then ca. 6.1 ml of formaldehyde were slowly added. After addition the reaction mixture was heated to 70°C and maintained at this temperature for about 30 minutes.

The reaction mixture was cooled to room temperature (20 - 30°C), ca. 27 ml of purified water were added and the mixture was stirred for 30 minutes. Then the reaction mixture was cooled to 0 - 5°C and stirred at this temperature for at least 2 hours. The product marbofloxacin formate (MBXBZ) was centrifuged and washed with 10 - 15 g of cooled (0 - 5°C) purified water. The product was spun dried and collected.

Wet product MBXBZ was added to the mixture of 67 ml of ethanol, 67 ml of methylene chloride and 16.2 ml of ammonia solution (ca. 25 0). If phases did not separate, additional 63 ml of methylene chloride and 33 ml of purified water were added. The pH of the water phase was adjusted to be between 7 and 9.5, preferable between 7.5 and 8.5. The mixture was agitated for approximately 15 minutes to 1 hour and then the layers were separated and both phases were subjected to in process control (IPC) analysis.

If IPC results showed that extraction was not complete, ca. 63 ml of methylene chloride were added to the water layer and the extraction was repeated until the IPC specification was met.

The organic phases were combined and ca. 6.8 mg of sodium sulphate anhydrous and optionally 0.4 mg of activated charcoal were added. The mixture was mixed for at least 30 minutes and filtered, then organic solvent was distilled off to obtain crude marbofloxacin.

### Purification of the crude Marbofloxacin

In an inert atmosphere 5 g of purified water, 12 g of ethanol 96 % and 4.3 g of toluene (ratio between the solvents was within the following ranges: ethanol : toluene : water : 1.8 - 2.8 : 1 : 1.1 - 1.2) were charged into a reactor and wet crude marbofloxacin (MBXCA) from the previous step was added under nitrogen. The mixture was slowly heated to reflux (70 - 80°C) until a clear solution was obtained. The solution was stirred for 0.5 hour under this temperature and then one half of the azeotrope solvent mixture (toluene : water : ethanol = 51 % : 6 % : 43 %) was evaporated. Then the remaining mixture was cooled slowly to 5°C (allowed interval is between 0 and 25 °C) with agitation (optionally 1 % mass of product of disodium-EDTA can be added). The mixture was mixed for 1 to 3 hours and the product was then isolated by centrifugation, washed with 13 g of ethanol, spun dry and collected. The product was dried at temperature 40 - 45°C, p < 100 mbar for 8 hour.

### Example 23

### Purification of Marbofloxacin

Marbofloxacin was dissolved in 20 parts by weight of water by addition of acetic acid. Marbofloxacin was completely dissolved at pH of 5.3. Active charcoal was added and the mixture was stirred overnight. The mixture was then filtered using activated charcoal filter. The pH of the filtrate was adjusted to 7.2 by use of KOH, the obtained suspension was stirred for 1 hour at room temperature and then the precipitated product was recovered. Marbofloxacin with a purity of 99,9% (HPLC area) was obtained.

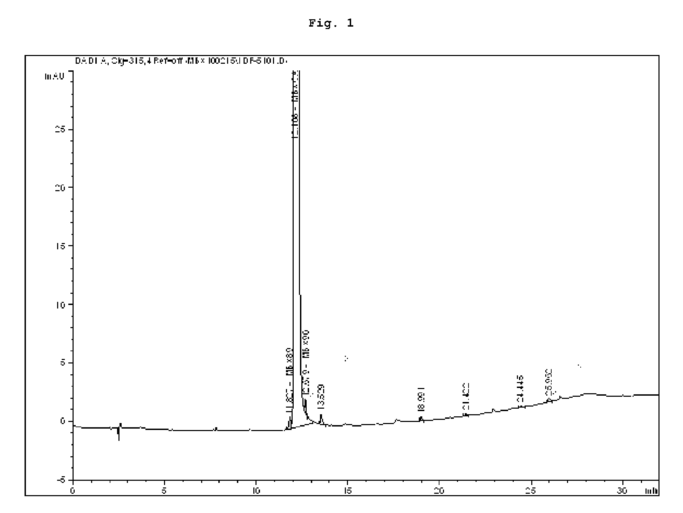

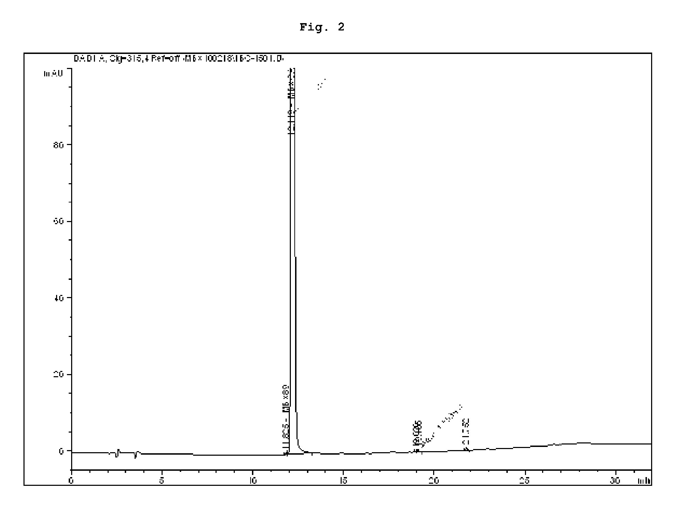

HPLC analysis was performed on a pentafluorophenyl propyl (PFP) column (type Luna® PFP, 150 x 4.6mm, 3µm, Phenomenex, USA); detector: UV315 nm; flow rate: 0.8 ml/min; injection volume: 5 µl; mobile phase: A: 0.02M NaH₂PO₄xH₂O+0,1% TEA, pH2.5; B: acetonitrile : methanol = 5:95 (v/v) ; gradient: 0'=10B, 25'=100B, 30'= 100B, 32'=10B. The HPLC chromatogram of marbofloxacin prior to purification is shown in Figure 1, the HPLC chromatogram after purification is shown in Figure 2. As evident from the chromatograms all products with retention time above 24min were successfully eliminated.

## Claims

1. A process for the preparation of a compound with formula I 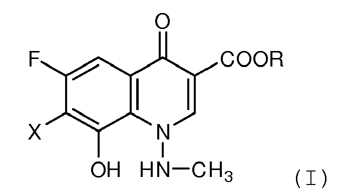 wherein
R is selected from H, alkyl, arylalkyl, alkali metal cation, NH₄ cation, NR¹R²R³R⁴cation,
R¹, R², R³ and R⁴ are independently selected from the group of H, alkyl, alkylaryl, aryl and/or heteroaryl,
X is halogen, such as chloro, bromo, fluoro; piperazinyl, which may be substituted or unsubstituted, preferably 4-methyl piperazinyl,
**characterized in that** it comprises reacting compound of formula II 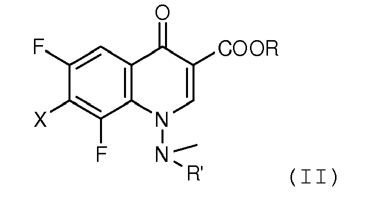
wherein R and X are as specified above and
R' is selected from H, formyl or COOAlkyl
with ammonium hydroxide of formula III
R¹R²R³R⁴NOH (III)
wherein R1, R2, R3, R4 are independently selected from the group of H, alkyl, alkylaryl, aryl and/or heteroaryl.

2. A process according claim 1, **characterized in that** compound of formula I wherein R and R' is H and X is 4-methylpiperazinyl, is prepared by reacting compound of formula II, wherein R is H or ethyl, R' is H and X is 4-piperazinyl, with tetramethylammonium hydroxide.

3. A process according to claim 1 or 2, **characterized in that** it is carried out in the neat conditions or in the absence of solvents.

4. A process according to any of the claims 1 to 3, **characterized in that** the ratio of compound of formula II to compound of formula III is 1:3 to 1:6.

5. A process according to any of the claims 1-4, **characterized in that** it comprises additional step of cyclizing compound of formula I with formic acid and formaldehyde to obtain compound of formula IV 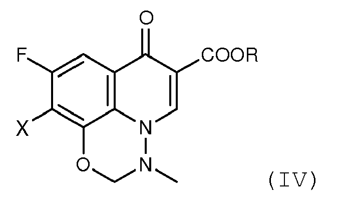 wherein R is H and X is piperazinyl, which may be substituted or unsubstituted, preferably 4-methyl piperazinyl.

6. A process for the one pot preparation of marbofloxacin or derivative thereof of formula IV 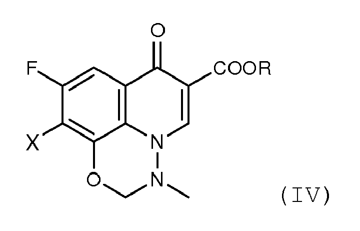 wherein R is H and X is piperazinyl, which may be substituted or unsubstituted, preferably 4-methyl piperazinyl,
**characterized in that** it comprises the following steps:
a) reacting compound of formula II 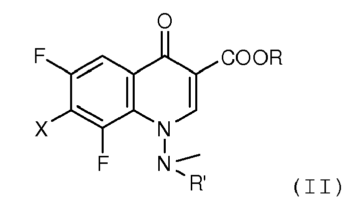 wherein
R is selected from the group of H, alkyl, arylalkyl, alkali metal cation, NH₄ cation, NR¹R²R³R⁴cation,
R¹, R², R³ and R⁴ are independently selected from the group of H, alkyl, alkylaryl, aryl and/or heteroaryl,
X is halogen, such as chloro, bromo, fluoro; piperazinyl, which may be substituted or unsubstituted, preferably 4-methyl piperazinyl, and
R' is selected from H, formyl, COOAlkyl,
with ammonium hydroxide of formula III
R¹R²R³R⁴NOH (III)
wherein R¹, R², R³ and R⁴ are as specified above,
b) reacting the obtained intermediate without recovery with formic acid and formaldehyde, and optionally
c) in case X is halogen its substitution by corresponding piperazine by conventional means to obtain after recovery compound of formula IV.

7. A process according to claim 6, **characterized in that** compound of formula II is used wherein R is selected from H, methyl, ethyl; X is 4-methyl piperazine and R' is H or formyl.

8. A process according to claim 6 or 7, **characterized in that** compound of formula II is used wherein R is H; X is 4-methyl piperazine and R' is H (formula IIa).

9. A process according to claims 6 to 8, **characterized in that that** step a) is performed in neat conditions or in the absence of solvent.

10. A process according to claims 6 to 9, **characterized in that** the ratio of compound of formula II to compound of formula III is 1:3 to 1:6.

11. The process of any one of claims 8 to 10, wherein the compound of formula (IIa) is obtained by hydrolysing a compound of formula (II) wherein R is C₁ to C₆ alkyl, R' is formyl and X is N-methylpiperazine (formula lie) with an acid or with a base, or by reacting a compound of formula (II) wherein R is H, R' is H and X is F (formula IId) with N-methylpiperazine.

12. The process of claim 11, wherein the compound of formula (IIc) is obtained by reacting a compound of formula (II) wherein wherein R is C₁ to C₆ alkyl, R' is formyl and X is F (formula IIb) with N-methylpiperazine.

13. The process of claim 11, wherein the compound of formula (IId) is obtained by hydrolysing a compound of formula (II) wherein R is C₁ to C₆ alkyl, R' is formyl and X is F (formula IIb) with an acid or with a base.

14. The process of claim 12 or 13, further comprising reacting compound (IIa) with ammonium hydroxide (III) to give the corresponding compound of formula (I), and cyclising compound (I) with HCOOH and HCHO to marbofloxacin (IV) .

15. A process for purifying marbofloxacin or a derivative, intermediate or precursor thereof comprising a piperazine group, comprising the steps of
a') dissolving marbofloxacin or a marbofloxacin derivative, intermediate or precursor thereof comprising a piperazine group in water in the presence of an acid,
b') optionally adding activated carbon to the mixture of step (a'),
c') filtering the mixture of step (a') or (b') through filter, preferably an activated charcoal filter,
d') precipitating the product of step (c') by addition of a basic reagent.

16. Marbofloxacin prepared according to the process of claim 15.

17. The marbofloxacin of claim 16 containing less than 0.01% (HPLC area) of dimeric impurities.
